# EUROPEAN PATENT APPLICATION

(11) **EP 3 037 987 A1**
(43) Date of publication of application: **29.06.2016**
(21) Application number: 14847849.8
(22) Date of filing: 19.09.2014
(51) Int. Cl.: G06F 17/30

(54) **CELL OBSERVATION INFORMATION PROCESSING SYSTEM, CELL OBSERVATION INFORMATION PROCESSING METHOD, CELL OBSERVATION INFORMATION PROCESSING PROGRAM, RECORDING UNIT INCLUDED IN CELL OBSERVATION INFORMATION PROCESSING SYSTEM, AND DEVICE INCLUDED IN CELL OBSERVATION INFORMATION PROCESSING SYSTEM**

(30) Priority: 26.09.2013 JP 2013200190
(71) Applicant: Olympus Corporation, Shibuya-ku Tokyo 151-0072 (JP)
(72) Inventor: TANIKAWA, Yohei, Tokyo 151-0072 (JP); IGA, Yasunobu, Tokyo 151-0072 (JP); TAKIMOTO, Shinichi, Tokyo 151-0072 (JP)
(74) Representative: Gunzelmann, Rainer
(86) International application number: PCT/JP2014/074839
(87) International publication number: WO 2015/046053

(57) **Abstract**

[Object] To provide a cell observation information processing system that enables a user to chronologically graph activity data on cells under culture in a short time without trouble.

[Measures for solution] Including a storing section 12 that assigns, to each of observation information sets each of which has been acquired via an observation information acquiring section 20 in response to an acquisition order at each time point to carry a cell observation result such as a cell image and activity data, an image tag, which forms a main tag, and information containing at least one of user identifying information, cell identifying information, information on date/time when the observation information set was acquired, information on user's work for cell maintenance, apparatus identifying information, and activity data variation information, which form subordinate tags subordinately associated with the image tag, and stores them into an archive section 13; an image selecting section 16; a search criterion generating section 17 that searches through the archive section using the selected image tag, retrieves a matched search-tagged observation information set, and automatically generates a search criterion on the basis of the subordinate tags assigned to the search-tagged observation information set; and a retrieving section 15 that searches through the archive section using the search criterion generated and retrieves, from the archive section, data containing activity data provided in the observation information set.

## Description

### TECHNICAL FIELD

The present invention relates to a cell observation information processing system, a cell observation information processing method, a cell observation information processing program, an archive section provided for the cell observation information processing system, and apparatuses provided for the cell observation information processing system, each of which is to be used for quality management of cells to be used for research of biological object upon use of observation information on the cells acquired by an observation information acquiring apparatus such as a microscope.

### BACKGROUND ART

In the fields where experiment and research using cells are to be made, users continue culturing cells on a daily basis and control, through observation of cell images via microscopes, quality of the cells to be used for the experiment and research.

In general, cells to be used for experiment and research are separated from the biological body and cultured upon being planted in a culture medium in a certain culture container, to be proliferated. These cells to be cultured are called primary culture. When cells in a primary culture are proliferated to a predetermined confluency or number, a part of the cells in the culture container of the primary culture are transplanted to a culture medium in another culture container, to be cultured and proliferated there. This is called passage of culture. The cells for experiment and research separated from the primary culture are called subculture.

In experiment and research using cells, users repeat passage of culture while proliferating and maintaining cells in the cell container of the primary culture, and use cells in a subculture separated from the primary culture subjected to passage.

Therefore, to improve accuracy of the experiment and research, it is desired that cells used for each time of experiment and research are kept in good culture conditions.

In the course of culturing cells, the cell quality may be varied.

Variation of the cell quality includes short-term variation, such as abnormality conspicuous at a single glance like contamination, and long-term variation of the order of several weeks. Regarding the long-term variation, it is difficult to recognize it only through a cell image acquired at one time point.

Conventionally, cell quality has been recognized by eye observation, through microscopes, of cells in culture containers by users. Finding variations of the cell quality has been entrusted to memory and sense of users. However, entrusting it to the users' memory and sense would hinder accurate recognition of a long-term variation of the cell quality, to lead to failure in enhancing accuracy of experiment and research. For accurate recognition of long-term variations of cell quality, it is necessary to make observation information at individual time points searchable by compiling them into a database.

Conventionally, there has been proposed an image processing apparatus that has an image database in which and through which observation data can be stored and searched, in the following Patent Document 1, for example.

The apparatus described in Patent Document 1 is configured so that it makes it possible to store, in a certain relational database, observation images containing images of biological specimens, upon assigning to them information on acquisition conditions at the times of capturing the observation images, such as photographing conditions and photographing date/time, to designate the information on acquisition conditions as a search criterion, and to choose and display images that are matched with the search criterion.

### PRIOR ART DOCUMENTS

### PATENT DOCUMENTS

Patent Document 1: US Patent Appln. Pub. No. 2006/0206482

### SUMMARY OF THE INVENTION

### PROBLEMS TO BE SOLVED BY THE INVENTION

For accurate recognition of a long-term variation of cell quality, it is desirable to retrieve observation data on objective cells of a plurality of time points ranging from the past up to date, and to relatively compare the retrieved observation data on the chronological basis.

As observation data a relative comparison of which is to be made on the chronological basis, it is most effective to use, in addition to image information, cell activity data indicating cell activity by a number of cells, a cell confluency, a morphology, a survival rate or so. If the activity data acquired at individual time points are graphed chronologically, user can easily check a long-term variation of cell quality.

However, Patent Document 1 merely discloses the configuration for storing, in a database, images and acquisition conditions upon linking them to each other. The invention described in Patent Document 1 has no conception of using the database for cell culturing or passage culturing. Therefore, when the apparatus described in Patent Document 1 is applied to management of cells that are used for experiments, there exist the problems shown below because of the circumstances peculiar to the field of experiment and research using cells.

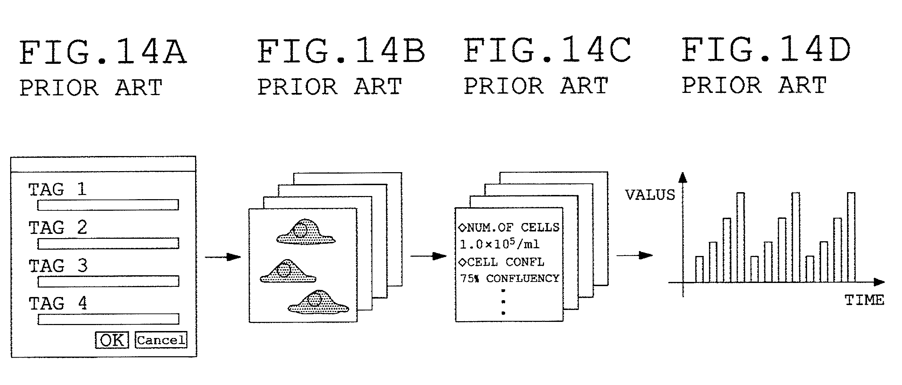

In order to retrieve observation data on objective cells of a plurality of time points ranging from the past up to date and to graph activity data chronologically on the basis of the retrieved observation data by applying the technique of the apparatus described in Patent Document 1 - storing, in a certain relational database, image information (observation images) upon assigning to them, as tags, information on photographing conditions, photographing date/time etc., and searching and retrieving desired images by appropriate combination of the tags-, it is necessary to follow the work procedure shown in FIGs. 14A-14D.

First, for example in a tag setting screen as shown in FIG. 14A, tags (information on photographing conditions, photographing date/time, etc.) in a database file as assigned to corresponding image information are individually set in tag item input fields (in the example of FIG. 14A, TAG 1 to TAG 4).

Then, a group of images stored in the database are searched and retrieved upon use of the set (combination of) tags. FIG. 14B is a diagram that schematically shows one example of the retrieved group of images.

Then, the individual images in the image group undergo image processing and analysis, and activity data in terms of the number of cells, cell confluency, etc. are calculated out. FIG. 14C is a diagram that shows one example of the activity data.

Then, as shown in FIG. 14D, the activity data as calculated out are rearranged in a chronological order, graphed.

According to the work procedure as shown in FIGs. 14A-14D, however, the work at each process is complicated, and thus not a small effort and time is spent until the activity data are rearranged in a chronological order and graphed.

In particular, in a case where a user wishes to review, in the midst of his or her working for passage, activity data on cells as chronologically graphed, a long time may not be given for preparation and display of a chronological graph of the activity data, for a harmful influence such as by stress exerted on the cells needs to be excluded as much as possible.

Further, in the tag setting screen as shown in FIG 14, if a user is to search and retrieve images from the database by setting a plurality of tags (information on photographing condition, photographing time/date, etc.) one by one, the operation is not only troublesome, but also liable to cause erroneous setting of tags.

In addition, in the case where a plurality of tags (information on photographing conditions, photographing time/date etc.) are to be individually set so that images are searched and retrieved from the database, at the stage where the user is just going to set tags in the tag setting screen shown in FIG. 14A, the cell setting screen dose not allow cell images to be displayed as related data. Therefore, the user cannot set tags while intuitively grasping images as the observation information desired to be searched for and retrieved.

A few aspects of the present invention are proposed to solve the above-described conventional problems; an object of the present invention is to provide a cell observation information processing system, a cell observation information processing method, a cell observation information processing program, an archive section provided for the cell observation information processing system, and an apparatus provided for the cell observation information processing system, each of which enables users to shorten the time taken to chronologically graph activity data of cells under culture by saving operation effort as much as possible.

Alternatively, according to a few aspects, an object of the present invention is to provide a cell observation information processing system, a cell observation information processing method, a cell observation information processing program, an archive section provided for the cell observation information processing system, and an apparatus provided for the cell observation information processing system, each of which allows users to make intuitive operation, upon preventing operation errors in chronologically graphing activity data of cells under culture.

### MEASURES TO SOLVE THE PROBLEMS

To attain the above-mentioned objects, a cell observation information processing system according to one aspect of the present invention includes: a storing section that assigns, to each of observation information sets each of which has been acquired via an observation information acquiring section in response to an acquisition order at each time point to carry a cell observation result such as a cell image, activity data indicating cell activity by a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date/time information for identifying a date/time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section; an image selecting section that lets a user select a desired image tag; a search criterion generating section that searches through the archive section using the image tag selected via the image selecting section, retrieves a search-tagged observation information set to which a search tag matched with the image tag has been assigned, and automatically generates a search criterion on the basis of subordinate tags, out of the search tags assigned to the search-tagged observation information set having been retrieved; and a retrieving section that searches through the archive section using the search criterion generated by the search criterion generating section and retrieves, from the archive section, data containing activity data provided in search-tagged observation information sets to which search tags matched with the search criterion have been assigned.

A cell observation information processing method according to another aspect of the present invention includes: a storing step for assigning, to each of observation information sets each of which has been acquired via an observation information acquiring section in response to an acquisition order at each time point to carry a cell observation result such as a cell image, activity data indicating cell activity by a number of cells, a cell confluency, a morphology, and a survival rate, and an observation title, search tags for searching for the observation information set, the search tags including an image tag, which forms amain tag, for indicating the cell image, and information containing at least one of user identifying information for identifying a user who conducts cell observation, cell identifying information for identifying cells under observation, date/time information for identifying a date/time when the observation information set was acquired, information on user's work for cell maintenance, apparatus identifying information for identifying an apparatus used in acquiring the observation information set, and activity data variation information, which form subordinate tags subordinately associated with the image tag, and storing each of search-tagged observation information sets, to each of which the search tags have been assigned, into an archive section; a search criterion generating step for letting a user select a desired image tag, searching through the archive section using the selected image tag, retrieving a search-tagged observation information set to which a search tag matched with the image tag has been assigned, and automatically generating a search criterion on the basis of subordinate tags, out of the search tags assigned to the search-tagged observation information set as retrieved; and a retrieving step for searching through the archive section using the search criterion generated at the search criterion generating step and retrieving, from the archive section, data containing activity data provided in search-tagged observation information sets to which search tags matched with the search criterion have been assigned.

A cell observation information processing program according to still another aspect of the present invention makes a computer function as: a storing means that assigns, to each of observation information sets each of which has been acquired via an observation information acquiring section in response to an acquisition order at each time point to carry a cell observation result such as a cell image, activity data indicating cell activity by a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date/time information for identifying a date/time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section; a search criterion generating means that lets a user select a desired image tag, searches through the archive section using the selected image tag, retrieves a search-tagged observation information set to which a search tag matched with the image tag has been assigned, and automatically generates a search criterion on the basis of subordinate tags, out of the search tags assigned to the search-tagged observation information set having been retrieved; and a retrieving means that searches through the archive section using the search criterion generated by the search criterion generating means and retrieves, from the archive section, data containing activity data provided in search-tagged observation information sets to which search tags matched with the search criterion have been assigned.

### BRIEF DESCRIPTION OF THE DRAWINGS

FIG. 1 is a block diagram that schematically shows the basic configuration of the cell observation information processing system according to the present invention.
FIG. 2 is an explanatory diagram that shows the configuration of the cell observation information processing system according to a first embodiment mode of the present invention.
FIG. 3 is an explanatory diagram that shows one example of data structure of search-tagged observation information sets stored by the storing section in the archive section in the cell observation information processing system of FIG. 2.
FIG. 4 is an explanatory diagram that schematically shows a relationship between the activity data and the image tag and other information, as one example of data structure of the essential part in a record of the observation information sets recorded in the archive section in the cell image observation processing system of FIG. 2.
FIG. 5 is an explanatory diagram that shows one configuration example of an image selecting screen that forms an image selecting section for making a user select an image tag in the cell observation information processing system of FIG. 2.
FIG. 6 is an explanatory diagram that shows the relationship between observation information sets acquired by the observation information acquiring section and acquisition time points in the cell observation information processing system of FIG.2.
FIG. 7 is a block diagram that shows one example of the procedure from tag selection by user to chronological display of activity data using the cell observation information processing system of FIG. 2.
FIG. 8 is an explanatory diagram that schematically shows the flow of the procedure from tag selection by user to chronological display of activity data using the cell observation information processing system of FIG. 2.
FIG. 9 is a flow chart that shows one example of the procedure from tag selection by user to chronological display of activity data using the cell observation information processing system of FIG. 2.
FIGs. 10A-10H are graphs that individually show display modes in which data containing activity data retrieved by use of the cell observation information processing system of FIG. 2 are chronologically displayed on a display surface; FIGs. 10A-10G show individual examples, and FIG. 10H is one example of display mode in which activity data at the date/time when an observation information set was acquired and activity data at the date/time when other observation information sets were acquired are distinguishingly displayed in the graph of FIG. 10A.
FIG. 11 is an explanatory diagram that shows the configuration of the cell observation information processing system according to one modified example of FIG. 2.
FIG. 12 is an explanatory diagram that shows the configuration of the cell observation information processing system according to another modified example of FIG. 2.
FIG. 13 is an explanatory diagram that shows the configuration of the cell observation information processing system according to still another modified example of FIG. 2.
FIGs. 14A-14D are explanatory diagrams that schematically show the flow of the operation procedure necessary for retrieving observation data on objective cells of a plurality of time points ranging from the past up to date and chronologically graphing activity data from the retrieved observation data by applying the search and retrieval technique of the apparatus described in Patent Document 1; where FIG. 14A is a diagram that schematically shows one example of tag setting screen at the stage of setting tags, FIG. 14B is a diagram that schematically shows one example of retrieved images at the stage of searching and retrieving images using the set tags; FIG. 14C is a diagram that schematically shows one example of activity data at the stage of calculating activity data from the individual retrieved images; and FIG. 14D is a diagram that schematically shows one example of chronological graph of the activity data at the stage of chronologically graphing the activity data.

### MODE FOR CARRYING OUT THE INVENTION

The embodiment mode of the present invention will be explained below. The embodiment mode explained below does not improperly limit the contents of the present invention recited in the claimed scope for a patent. In addition, not all of the configurations explained in reference to the embodiment mode below are indispensable configurations for the present invention.

A cell observation information processing system according to the embodiment mode of the present invention includes: a storing section that assigns, to each of observation information sets each of which has been acquired via an observation information acquiring section in response to an acquisition order at each time point to carry a cell observation result such as a cell image, activity data indicating cell activity by a number of cells, a cell confluency, a morphology, and a survival rate, and an observation title, search tags for searching for the observation information set, the search tags including an image tag, which forms amain tag, for indicating the cell image, and information containing at least one of user identifying information for identifying a user who conducts cell observation, cell identifying information for identifying cells under observation, date/time information for identifying a date/time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section; an image selecting section that lets a user select a desired image tag; a search criterion generating section that searches through the archive section using the image tag selected via the image selecting section, retrieves a search-tagged observation information set to which a search tag matched with the image tag has been assigned, and automatically generates a search criterion on the basis of subordinate tags, out of the search tags assigned to the search-tagged observation information set having been retrieved; and a retrieving section that searches through the archive section using the search criterion generated by the search criterion generating section and retrieves, from the archive section, data containing activity data provided in search-tagged observation information sets to which search tags matched with the search criterion have been assigned.

The image tag is a tag for indicating a cell image, to be specific, an image displayable in a small size in an input screen or information containing an image and its corresponding name integrally combined together.

If the configuration is made so that, as in the cell observation information processing system according to the embodiment mode of the present invention, the search criterion generating section searches through the archive section using an image tag selected by the user via the image selecting section, retrieves a search-tagged observation information set to which a search tag matched with the image tag has been assigned, and automatically generates a search criterion on the basis of subordinate tags, out of the search tags assigned to the search-tagged observation information set having been retrieved, and so that the retrieving section searches through the archive section using the search criterion generated by the search criterion generating section and retrieves, from the archive section, data containing activity data provided in search-tagged observation information sets to which search tags matched with the search criterion have been assigned, only by an operation for selecting an image tag in the image selecting section, the user can set, in bulk, a plurality of information items that are to be search tags, as subordinate tags subordinately associated with the image tag; it is not necessary to set a plurality of tags one by one as in the case where the configuration described in Patent Document 1 is adopted. In addition, since activity data provided in the observation information sets stored in the archive section are retrievable on the basis of subordinate tags, the user can be saved from the trouble of calculating activity data each time an image is retrieved as in the case where the configuration described in Patent Document 1 is adopted. The retrieved activity data can be graphed and displayed on a display surface upon being rearranged in order of date/time when the observation information sets were acquired. By making the display surface chronologically display the activity data, the user can instantly grasp the chronological variation of the activity data in association with the cell image indicated by the image tag selected from the image selecting section.

Therefore, according to the cell observation information processing system of the embodiment mode of the present invention, the user is enabled to shorten the time taken to chronologically graph activity data of cells under culture by saving operation effort as much as possible, and is allowed to make intuitive operation without operation errors.

Regarding the subordinate tags, on the basis of which a search criterion is automatically generated by the search criterion generating section and which are subordinately associated with the image tag, an appropriate combination of items such as user identifying information, date/time information for identifying a date/time when an observation information set was acquired, cell identifying information (cell name, passage number) for identifying the cells as an observation target is preferable.

According to the embodiment mode of the present invention, in the situation where the search criterion is automatically generated on the basis of date/time information for identifying a date/time when an observation information set was acquired, the search criterion generating section creates, as a search criterion, a time range having a predetermined time width respectively before and after the designated date/time information as a reference.

According to the embodiment mode of the present invention, in addition to the effects described above, the following incidental effects are achieved in accordance with a search criterion automatically generated by the search criterion generating section.

### (1) Function and effect of the configuration in which search is made by use of a tag of user identifying information (user oneself or acting worker)

As stated above, the cell observation information processing system of the embodiment mode of the present invention is configured so that the user identifying information can be automatically generated as a search criterion.

The user identifying information is identifying information for identifying the user who conducts cell observation. To be specific, this is identifying information for identifying the directly concerned user who inputs cell observation result or an acting person who conducts cell observation or inputs cell observation result in place of the directly concerned user.

If the configuration ismade so that the user identifying information can be automatically generated as a search criterion, as in the cell observation information processing system according to the embodiment mode of the present invention, a user can retrieve observation data on cells under his or her management alone, while excluding observation data under management by others, a reference to which is not necessary, from the search result. As a result, the retrieval efficiency is improved and the time taken for cell quality checking can be shortened.

In particular, in the situation where quality checking of cells is conducted while the cells are taken outside the incubator, the shortened time for checking cell quality can moderate damage given to the cells.

The configuration in which the user identifying information can be automatically generated as a search criterion is useful in a situation where a user, upon logging in the installation, checks, in bulk, observation data on cells under his or her management alone that were acquired at a plurality of time points, for example in the case where a user reviews past observation data on cells under his or her management, before he or she conducts the work of cell quality checking of the day.

### (2) Function and effect of the configuration in which search is made by use of tags of cell identifying information (cell name, cell kind, cell level information (either "primary culture" or "subculture"), passage number)

As described above, the cell observation information processing system according to the embodiment mode of the present invention is configured so that cell identifying information can be automatically generated as a search criterion.

The cell identifying information is identifying information for identifying cells that are an observation target. To be specific, it is identifying information that includes at least one of: a name of the cells, a kind of the cells, cell level information for identifying whether the cells are of the primary culture to be cultured or of a subculture which has been separated from and cut out of the primary culture, and passage number of the cells.

A cell name is a name freely given by a user to the cells used for experiment and research, and a cell kind is a specific category into which the cells are classified.

The cell observation information processing system according to the embodiment mode of the present invention is preferably configured so that the search criterion generating section automatically generates, as search a criterion, information at least containing the cell identifying information.

### (2-1) Function and effect of the configuration for search by cell name

A cell name is a name freely given by each user to cells of a primary culture and its subculture used for experiment and research.

If the configuration is made so that a cell name can be automatically generated as a search criterion, as in the cell observation information processing system according to the embodiment mode of the present invention, observation data on cells of a primary culture and its subculture are retrievable in bulk. As a result, a user can check a long-term variation of the quality of the cells under culture.

In the case of handling cells that are cultured without passage, for example, the configuration for automatically generating a cell name as a search criterion is useful for checking a long-term variation of the quality of the cells under culture.

### (2-2) Function and effect of the configuration for search by cell name and cell level information (either of "primary culture" or "subculture")

If the configuration is made so that a cell name and cell level information ("primary culture" or "subculture") can be automatically generated as a search criterion, as in the cell observation information processing system according to the embodiment mode of the present invention, observation data on cells limited to those with a certain cell name and a desired cell level ("primary culture" or "subculture") are retrievable in bulk. As a result, a user can improve efficiency of retrieval while checking a long-term variation of the cells, the cell level of which is either one of primary culture and subculture.

The configuration for acquiring a cell name and cell level information (either "primary culture" or "subculture") as a search criterion is particularly useful for sorting cells into cut-out cells (subculture) and a culture from which the cells are cut out (primary culture) and checking a long-term variation of quality of the cells having a cell level that is either primary culture or subculture.

In general, long-term variations of cell quality would proceed slowly. Therefore, for comparison of observation data, observation data on a primary culture, which have been recorded continually and extensively since commencement of culture, are more convenient than observation data on a subculture, which were recorded as a little amount just before a down stream experiment.

Therefore, according to the cell observation information processing system of the embodiment mode of the present invention, automatic generation of cell level information (primary culture or subculture) as a search criterion facilitates efficient retrieval of observation data that are particularly useful for recognizing cell quality, and accordingly can shorten the time taken for checking the cell quality by the user.

### (2-3) Function and effect of the configuration for search by cell name, cell level information (either "primary culture" or "subculture"), and passage number

If the configuration is made so that a cell name, cell level information (either "primary culture" or "subculture"), and a passage number (in addition to direct reference to a particular generation such as "3rd generation", a range of generation such as "2nd and later generations" and "5th and former generations" is available) can be automatically generated as a search criterion, as in the cell observation information processing system according to the embodiment mode of the present invention, a user can improve efficiency of retrieval while checking a long-term variation of cells, by limiting observation data on cells under passage culture to those on cells with a certain cell name and a certain cell level ("primary culture" or "subculture") of certain generations.

The configuration for automatically generating a cell name, cell level information (primary culture or subculture) and a passage number as a search criterion is particularly useful for checking a long-term variation over generations (passage number) of quality of cells that are subject to work for passage of culture.

### (2-4) Function and effect of the configuration for search by user identifying information and cell name

Users would individually manage naming of cells. Therefore, cell names given by a plurality of users may happen to coincide with each other.

Under such a cell management situation where a plurality of users use an identical cell name, if user identifying information is not automatically generated as a search criterion, observation data having an identical cell name but of a different user would be mixed in the retrieved result.

Therefore, if the configuration is made so that user identifying information and a cell name can be automatically generated as a search criterion, as in the cell observation information processing system according to the embodiment mode of the present invention, a user can retrieve only the observation data as intended even if an identical cell name is given by a plurality of users.

The configuration for automatically generating user identifying information and a cell name as a search criterion is useful, for example in a case of handling cells that are cultured without passage, for checking a long-term variation of quality of the cells in a situation where a plurality of users manage naming of observation data.

In addition, in the cell observation information processing system of the embodiment mode of the present invention, it is also possible to conduct a search upon using "user identifying information, cell name, and cell level information (primary culture or subculture)", "user identifying information, cell name, cell level information (primary culture or subculture), and passage number", "user identifying information, cell name, cell level information (primary culture or subculture), and variation of activity data", or "user identifying information, cell name, and passage number" as a criterion.

### (3) Function and effect of the configuration in which search is made by use of activity data variation information

As stated above, the cell observation information processing system according to the embodiment mode of the present invention is configured so that activity data variation information can be automatically generated as a search criterion.

The activity data variation information is expressed, for example by an amount of variation (thatis, a rate of variation) between respective activity data (for example, cell confluency, number of cells or survival rate) at a plurality of time points, and can be shown, for example by a slope of a plotted line in a graph.

For example in a graph that shows variation information on number of cells, it is probable that cells in a certain period of observation information set acquisition that corresponds to a part of the graph where the variation rate of the number of cells is large (the slope of the plotted line is steep) are more vigorously proliferated than cells during a period of observation information set acquisition that corresponds to a part where the variation rate of the number of cells is small (the slope of the plotted line is moderate).

Therefore, if the configuration is made so that activity data variation information can be automatically generated as a search criterion as in the cell observation information processing system according to the embodiment mode of the present invention, observation data on cells that are vigorously proliferated are solely retrievable. As a result, upon selecting cells of an observation information set acquisition period that is found out from the observation data as a period during which cells are vigorously proliferated, users can use the cells of this observation information set acquisition period alone for a down stream experiment, to raise success rate of the down stream experiment.

In addition, the cell observation information processing system according to the embodiment mode of the present invention is preferably configured as follows.

An observation information set is preferably constructed of information that provides a plurality of observation results corresponding to image capture at one time point directed to a plurality of observation positions on a specimen containing the cells.

The information providing a plurality of observation results preferably contains a plurality of cell images.

Alternatively, the information providing the plurality of observation results preferably contains a value calculated on the basis of a plurality of cell images.

Alternatively, the information providing the plurality of observation results preferably contains a plurality of cell images and a value calculated on the basis of the plurality of cell images.

Alternatively, the information providing the plurality of observation results preferably contains a plurality of cell images, and the search tags include a value calculated on the basis of the plurality of cell images.

Alternatively, the information providing the plurality of observation results preferably contains a plurality of cell images, and the search tags include an image tag for displaying the plurality of cell images.

Alternatively, the information providing the plurality of observation results preferably contains a plurality of cell images, and the search tags include a value calculated on the basis of the plurality of cell images.

Alternatively, the information providing the plurality of observation results preferably contains a plurality of cell images, and the search tags include an image tag for displaying the plurality of cell images and a value calculated on the basis of the plurality of cell images.

Such configuration allows the user to more accurately grasp the cell quality even if the cells are unevenly distributed in a container.

As the value calculated on the basis of the plurality of cell images, for example an average of cell activity data in terms of cell confluency, number of cells, survival rate or so is applicable.

It is preferred that the cell observation information processing system according to the embodiment mode of the present invention further includes a presenting section that presents to a user data containing activity data provided in search-tagged observation information sets retrieved by the retrieving section upon rearranging them chronologically on the basis of date/time when the observation information sets were acquired.

If such a presenting section is provided, a chronological graph of activity data on cells under culture is displayed in a short time without trouble only by selection of an image tag, and thus the user can instantly grasp the conditions of chronological variation of the activity data by visually confirming the selected cell image and the chronological graph of activity data as directly associating them with each other.

Further, in the cell observation information processing system according to the embodiment mode of the present invention, it is preferred that the presenting section is provided with a distinguishing display processing part for displaying, among a plurality of activity data sets to be presented, the activity data of the date/time when the observation information set corresponding to the selected image tag was acquired in a display manner different from activity data of the date/time when the remaining observation information sets were acquired, such as the emphasized display manner and the marker-overlaid display manner.

In this way, if such a distinguishing display processing part is provided, the position of the activity data of the date/time when the observation information set corresponding to the user-selected image tag was acquired is made conspicuous in a graph that chronologically shows activity data of a plurality of time points, for example. Therefore, in a situation where the presenting section is to display the cell image corresponding to the image tag along with the chronological graph of the activity data, the user can easily check the cell image and the activity data as associating them with each other.

Hereinafter, an explanation is made on the embodiment mode of the present invention in reference to the drawings.

### First embodiment mode

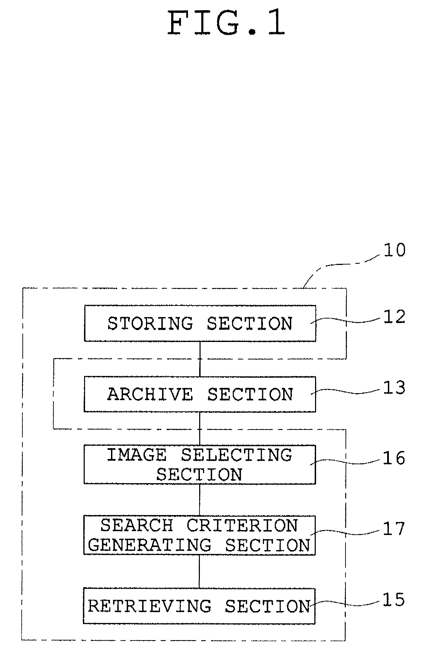

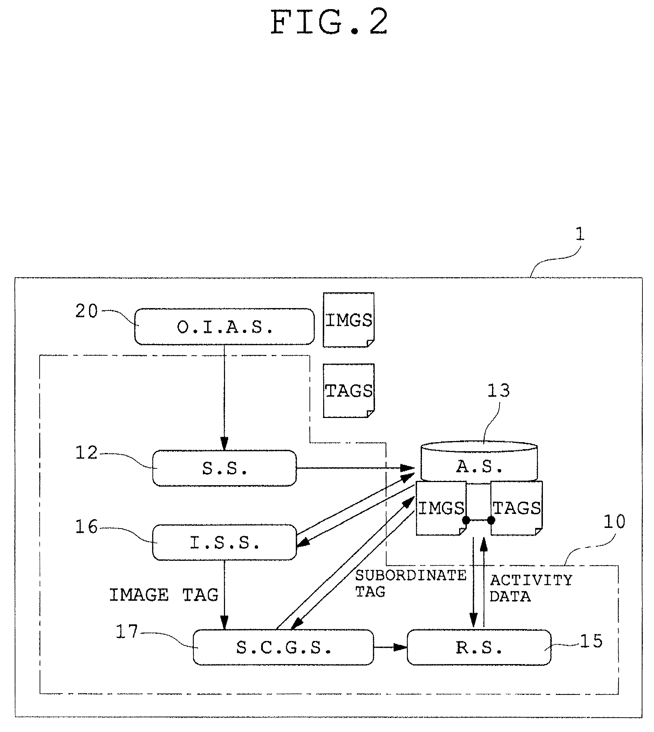

FIG. 1 is a block diagram that schematically shows the basic configuration of the cell observation information processing system according to the present invention. FIG. 2 is an explanatory diagram that shows the configuration of the cell observation information processing system according to a first embodiment mode of the present invention.

The cell observation information processing system 10 of the first embodiment mode includes, as shown in FIG. 1 and FIG. 2, a storing section 12, an image selecting section 16, a search criterion generating section 17 and a retrieving section 15. In FIG. 2, the reference numeral 1 denotes a cell observation information processing installation, the reference numeral 13 denotes an archive section, and the reference numeral 20 denotes an observation information acquiring section. In FIG. 2, the cell observation information processing system 10 is configured as a stand-alone system with the storing section 12, the image selecting section 16, the search criterion generating section 17 and the retrieving section 15 being provided in the single installation 1 together with the archive section 13 and the observation information acquiring section 20. The cell observation information processing installation 1 is configured, for example of a microscope apparatus provided with an image capture apparatus and a computer provided with a database.

The storing section 12 is configured to assign, to each of observation information sets each of which has been acquired via the observation information acquiring section 20 in response to an acquisition order at each time point, search tags for searching for the observation information set, the search tags including an image tag, which forms a main tag, for indicating a cell image, and information containing at least one of user identifying information, cell identifying information, date/time information for identifying the date/time when the observation information was acquired, information on user's work for cell maintenance, apparatus identifying information, and activity data variation information, which form subordinate tags subordinately associated with the image tag, and stores each of search-tagged observation information sets, to each of which the search tags have been assigned, into the archive section.

The observation information set, the user identifying information, the cell identifying information, the information on user's work for cell maintenance, and the apparatus identifying information are explained above.

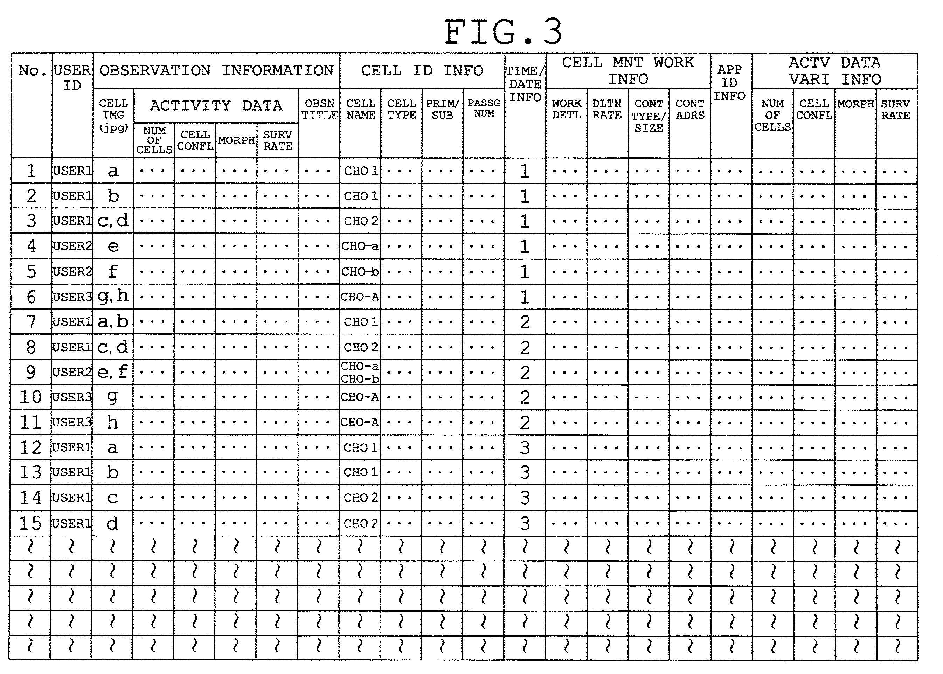

The archive section 13 has, for example as shown in FIG. 3, one or more records of the search-tagged observation information. Each record is created corresponding to image capture of a cell image at one time point, where observation information corresponding to the image capture of a cell image at one time point is interrelated with search tags for retrieving the observation information.

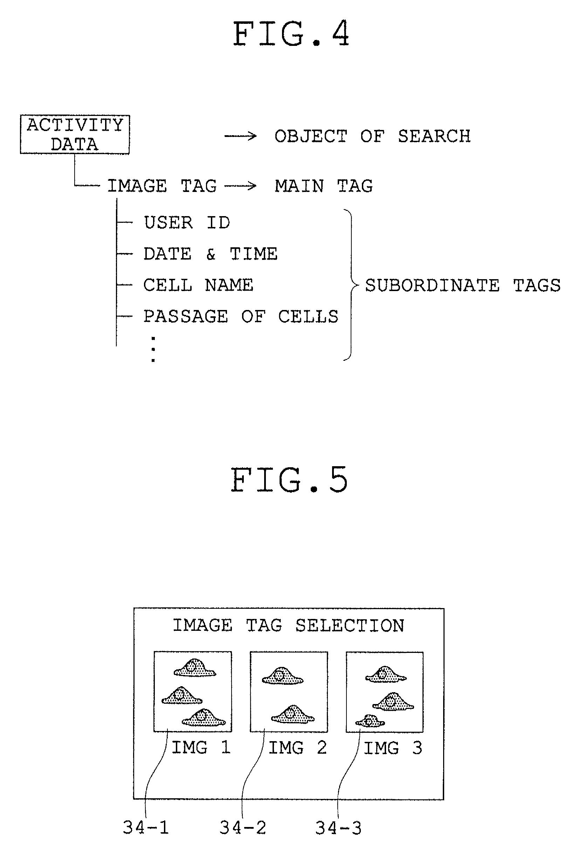

FIG. 4 is an explanatory diagram that schematically shows a relationship between the activity data and the image tag with other tags, as one example of data structure of the essential part in a record of the observation information sets recorded in the archive section 13 in the cell image observation processing system of FIG. 2.

In the example of FIG. 4, the image tag is made to correspond to the activity data in the observation information set. A group of information items including the user ID as the user identifying information, the date/time information, the cell name, the passage number etc. are associated, as subordinate tags, with the image tag. The configuration is made so that, when a search is made through the archive section 13 in the later-described search criterion generating section 17 with the image tag as a main tag, the group of information items including the user ID, the date/time information, the cell name, the passage number etc. which are subordinated to the image tag, are retrieved in bulk as subordinate tags.

While the user ID is used as the user identifying information in this embodiment mode for convenience' sake, the user identifying information is not necessarily limited to the user ID; any letters or symbols that can distinguish a certain user from other users, such as a name or nickname of the user, may work.

Although the data structure of a record of observation information recorded in the archive section in the FIG. 4 example does not show the cell kind as the cell identifying information, it may be configured so that the cell kind is associated as one of the subordinate tags, as a matter of course. Further, the data structure may be configured to associate any combination of items of desired subcategories under the user identifying information, the cell identifying information, the date/time information for identifying the date/time when the observation information set was acquired, the information on user's work for cell maintenance, the apparatus identifying information, and the activity data variation information (for example, cell name, cell kind, subculture/primary culture, passage number etc. in the cell identifying information) with the image tag, as a matter of course.

The image selecting section 16 is configured to let the user select a desired image tag, for example in an image tag selecting screen as shown in FIG. 5.

In FIG. 5, the reference numerals 34-1 to 34-n (in FIG. 5, n = 3 for convenience' sake) denote image tags. Each of the image tags 34-1 to 34-n has a form of a cell image and an image name integrally combined together and is displayed on the screen to be selectable for input.

The search criterion generating section 17 is configured to search through the archive section 13 using the image tag selected via the image selecting section 16, to retrieve a search-tagged observation information set to which a search tag matched with the image tag has been assigned, and to automatically generate a search criterion on the basis of subordinate tags, out of the search tags assigned to the search-tagged observation information set as retrieved.

The search criterion generation section 17 searches through the archive section 13 with an image tag, as the main tag, selected by the user for example among the image tags 34-1 to 34-n in the image tag selecting screen shown in FIG. 5, and retrieves a search-tagged observation information set to which a search tag matched with the image tag is assigned. Then, the search criterion generating section 17 automatically generates a search criterion on the basis of subordinate tags subordinately associated with the image tag (in the example of FIG. 4, a group of information formed of user ID, time/date information, cell name, and passage number, for example), out of the search tags assigned to the search-tagged observation information set as retrieved.

The retrieving section 15 is configured to search through the archive section 13 using the search criterion acquired by the search criterion generating section 17 and to retrieve, from the archive section 13, data containing activity data provided in the search-tagged observation information to which search tags matched with the search criterion has been assigned.

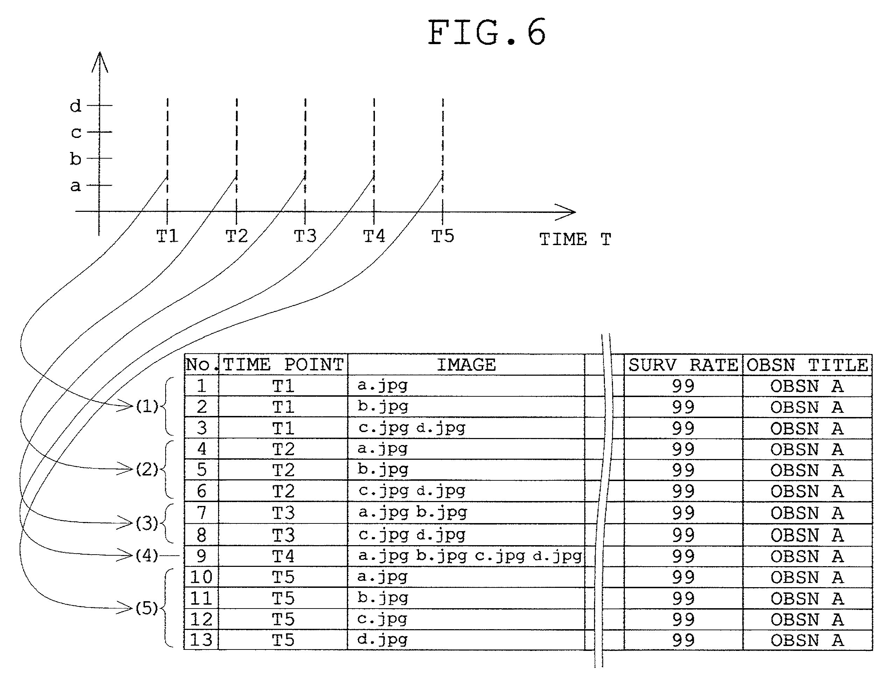

The observation information acquiring section 20 is configured to acquire an observation information set in response to an acquisition order at one time point. To be specific, the observation information acquiring section 20 is actuated when a user inputs an observation name and pushes an order button for observation information acquisition on an image-capture order screen (not shown in the drawings), makes an image capture apparatus (not shown in the drawings) provided in the installation 1 image-capture a specimen containing cells placed at an observation position, detects activity data that show cell activity in terms of cell confluency, morphology, survival rate or so by subjecting a cell image as captured to image processing and analysis processing via an image processing section and an image analyzing section (not shown in the drawings), and, as shown in FIG. 6, temporally stores, in a storage area (not shown in the drawings) in the installation 1, the cell image, the activity data and the observation name as one observation information set. In the case of FIG. 6 example, a group (1) of records numbered 1 to 3 form one observation information set acquired by the observation information acquiring section 20 in response to an acquisition order at a time point T1. Similarly, a group (2) of records numbered 4 to 6, a group (3) of records numbered 7 and 8, a group (4) of record numbered 9, and a group (5) of records numbered 10 to 13 form individual observation information sets acquired by the observation information acquiring section 20 in response to acquisition orders at different time points T2 to T5, respectively.

The cell observation information processing installation 1 is provided with a system log-in screen (not shown in the drawings). If a user inputs a log-in ID and a password of the system in the system log-in screen, a user-ID input screen (not shown in the drawings) is displayed. In the user-ID input screen, if the user inputs, by selection, his or her user ID as the user identifying information, the user ID is temporally stored in the storage area (not shown in the drawings) in the installation 1 and an image capture order screen (not shown in the drawings) is displayed so that the user can perform operation for image-capture order. The user-ID input screen (not shown in the drawings) may be configured to function as a system log-in screen also by letting a user perform input operation for a log-in ID and a password of the system together with input operation by selection for a user ID.

The cell observation information processing installation 1 may be provided with a tag acquiring section not shown in the drawings.

To be specific, the tag acquiring section is configured to acquire, as search tags, for example a user ID as user identifying information selected and inputted by a user in a user-ID input screen (not shown in the drawings) provided in the cell observation information processing installation 1 and for example cell level information ("primary culture" or "subculture"), a cell name and a passage number inputted by the user in a search tag input screen (not shown in the drawings) provided in the cell observation information processing installation 1. The tag acquiring section may be configured to automatically acquire preset values of the cell level information ("primary culture" or "subculture"), the name and the passage number without via input by the user in the search tag input screen (not shown in the drawings).

Also, the tag acquiring section acquires image capture date/time having been recorded as a log in cell images in each observation information set acquired via the observation information acquiring section 20, as date/time information for identifying the date/time when the observation information set was acquired. Also, the tag acquiring section acquires an image tag from the cell images in each observation information set acquired via the observation information acquiring section 20.

The configuration may be made so that the storing section 12 is provided with these functions of the tag acquiring section.

Further, the cell observation information processing system 10 is preferably provided with a display surface (not shown in the drawings) as a presenting section for presenting data containing search-tagged observation information retrieved by the retrieving section 15.

An explanation is made on the processing procedure from user's tag selection to chronological display of activity data, using the cell observation information processing system according to the first embodiment mode thus configured.

The procedure of quality management of cells cultured by users is divided into a stage of storing observation information sets on the cells under culture into the archive section 13 and a stage of searching and retrieving desired observation information sets on cells from the archive section 13 and checking quality of the cells. The procedure from user's tag selection to chronological display of activity data is performed at the stage of searching and retrieving desired observation information sets on cells from the archive section 13 and checking quality of the cells.

First, the processing procedure at the stage of storing observation information sets on cells under culture into the archive section is briefly explained.

The user inputs a log-in ID and a password of the system in the system log-in screen (not shown in the drawings). Then, the user ID input screen (not shown in the drawings) is displayed. Then, the user selects and inputs his or her own user ID. Consequently, the user ID is temporally stored in the storage area in the installation 1 and an image-capture order screen (not shown in the drawings) is displayed, to allow the user to perform operation for image-capture order.

Then, in the image-capture order screen (not shown in the drawings), the user inputs an observation name and pushes an order button for ordering acquisition of an observation information set. Then, the observation information acquiring section 20 makes the image capture apparatus (not shown in the drawings) image-capture a specimen containing cells placed at the observation position, detects activity data that shows cell activity in terms of number of cells, cell confluency, morphology, survival rate or so, for example by subjecting a cell image as captured to image processing and analysis processing via the image processing section and the image analyzing section (not shown in the drawings), and temporally stores, in the storage area (not shown in the drawings) in the installation 1, the cell image, the activity data and the observation name as one observation information set. In this way, the observation information set at one time point is acquired. It is noted that acquisition of the observation information set can be repeated a plurality of times for different acquisition time points.

Upon completion of input and observation information acquisition order by the user from the image-capture order screen and acquisition of the observation information set by the observation information acquiring section 20, the user inputs "primary culture" or "subculture", a cell name and a passage number in the search-tag input screen (not shown in the drawings). When the input completes, the tag acquiring section (not shown in the drawings) acquires, as search tags, "primary culture" or "subculture", the cell name and the passage number, which have been inputted by the user in the tag input screen, and the user ID, which has been inputted by the user in the user-ID selectable input screen. In the above-explained example, the configuration is made so that, after a plurality of times of repetition of observation information acquisition by the observation information acquiring section 20, the presenting section (not shown in the drawings) is switched to the mode for inputting tags in the search-tag input screen (not shown in the drawings) and acquiring the search tags by the tag acquiring section (not shown in the drawings). However, the configuration may be made so that the presenting section is switched each time after an observation information set is acquired by the observation information acquiring section, to the mode for inputting tags in the search-tag input screen (not shown in the drawings) and acquiring the search tags by the tag acquiring section (not shown in the drawings). Regarding timing of search tag assignment, the configuration may be made so that the presenting section is switched to the mode for inputting tags in the search tag input screen (not shown in the drawings) and acquiring the search tags by the tag acquiring section (not shown in the drawings) in real time after acquisition of an observation information set by the observation information acquiring section 20, or so that the presenting section is switched to the mode for inputting tags in the search-tag input screen (not shown in the drawings) and acquiring the search tags by the tag acquiring section (not shown in the drawings) a certain time after acquisition of the observation information set by the observation information acquiring section 20.

After that, the storing section 12 assigns, to each observation information set acquired in response to an acquisition order at each time point, as search tags for example including those acquired by the tag acquiring section, an image tag, which forms a main tag, and information containing at least one of user identifying information, cell identifying information, date/time information, information on user's work for cell maintenance, apparatus identifying information, and activity data variation information, which form subordinate tags subordinately associated with the image tag, and then stores the search-tagged observation information set, to which the search tags have been assigned, into the archive section 13. It is noted that assignment of search tags to the observation information set by the storing section 12 may be made, after acquisition of the observation information set by the observation information acquiring section 20, by associating predetermined subordinate tags to the image tag without intervention of the tag acquiring section, as a matter of course. Regarding timing of search tag assignment by the storing section 12 in this case, the configuration may be made so that the storing section 12 associates predetermined subordinate tags with the image tag in real time after acquisition of an observation information set by the observation information acquiring section 20, or so that the storing section 12 associates predetermined subordinate tags with the image tag a certain time after acquisition of the observation information set by the observation information acquiring section 20.

Upon completion of assignment of tags to observation information set at every time point and storage of the search-tagged observation information into the archive section 13, the procedure at the stage of storing observation information sets on cells under culture into the archive section 13 finishes.

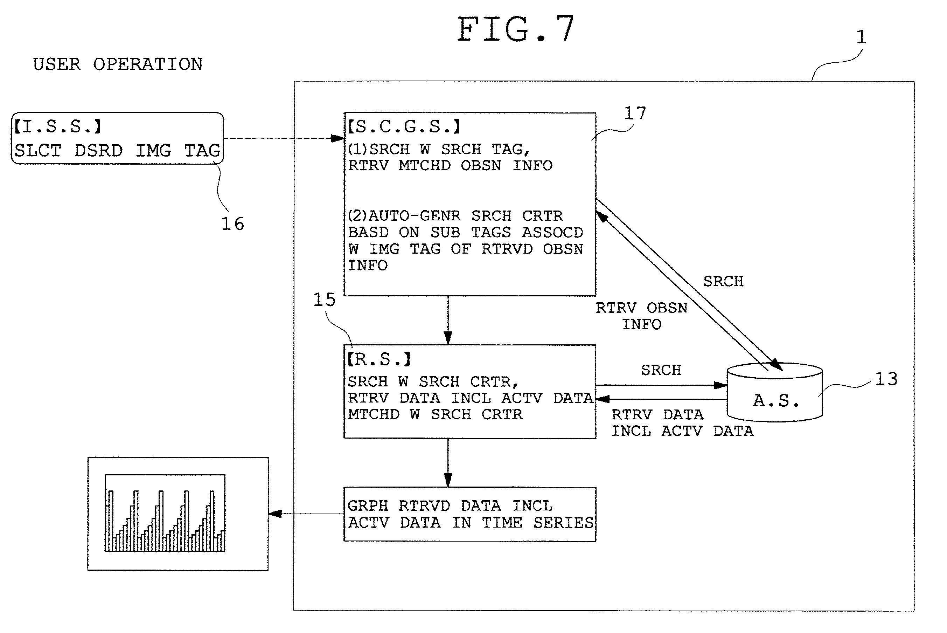

Next, the processing procedure at the stage of searching and retrieving desired observation information sets on cells from the archive section 13 is explained in reference to FIG. 7 to FIG. 9.

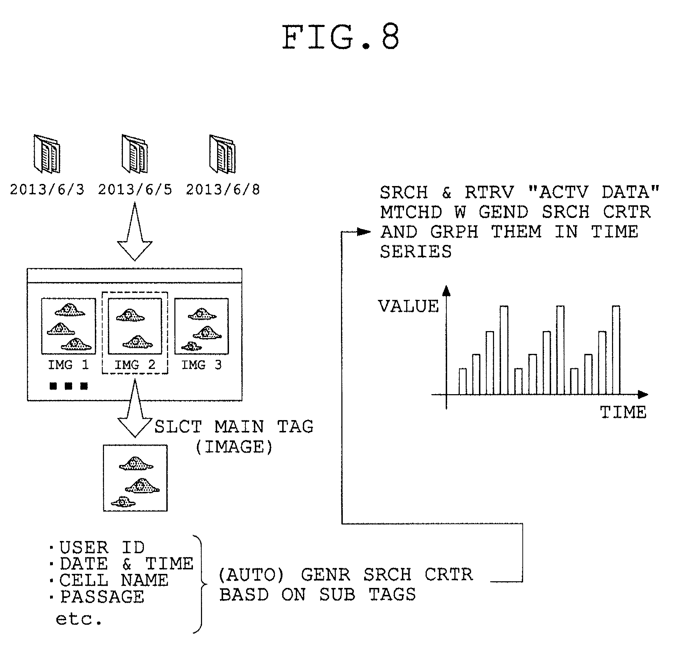

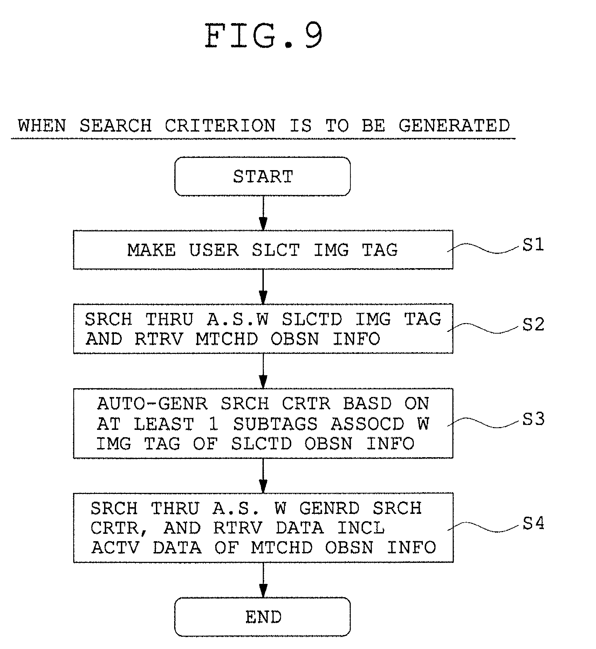

FIG. 7 is a block diagram that shows one example of the procedure from user's tag selection to chronological display of activity data using the cell observation information processing system of FIG. 2. FIG. 8 is an explanatory diagram that schematically shows the flow of the procedure from user's tag selection to chronological display of activity data using the cell observation information processing system of FIG. 2. FIG. 9 is a flow chart that shows one example of the procedure from user's tag selection to chronological display of activity data using the cell observation information processing system of FIG. 2.

In the image tag selecting screen as shown in FIG. 5, the user designates a desired image tag (Step S1).

Then, the search criterion generating section 17 searches through the archive section 13 with, as a main tag, an image tag selected by the user among image tags 34-1 to 34-n on the image tag selecting screen shown in FIG. 5, and retrieves a search-tagged observation information set to which a search tag matched with the image tag has been assigned (Step S2).

Then, the search criterion generating section 17 automatically generates a search criterion on the basis of subordinate tags (in the example of FIG. 5, a group of information items including user ID, date/time information, cell name, passage number etc) subordinately associated with the image tag, among the search tags assigned to the search-tagged observation information set having been retrieved (Step S3).

Next, the retrieving section 15 searches through the archive section 13 using the search criterion automatically generated by the search criterion generating section 17, and retrieves data containing activity data provided in the search-tagged observation information set to which search tags matched with the search criterion have been assigned (Step S4). Whereby, the procedure from user's tag selection to chronological display of activity data is completed.

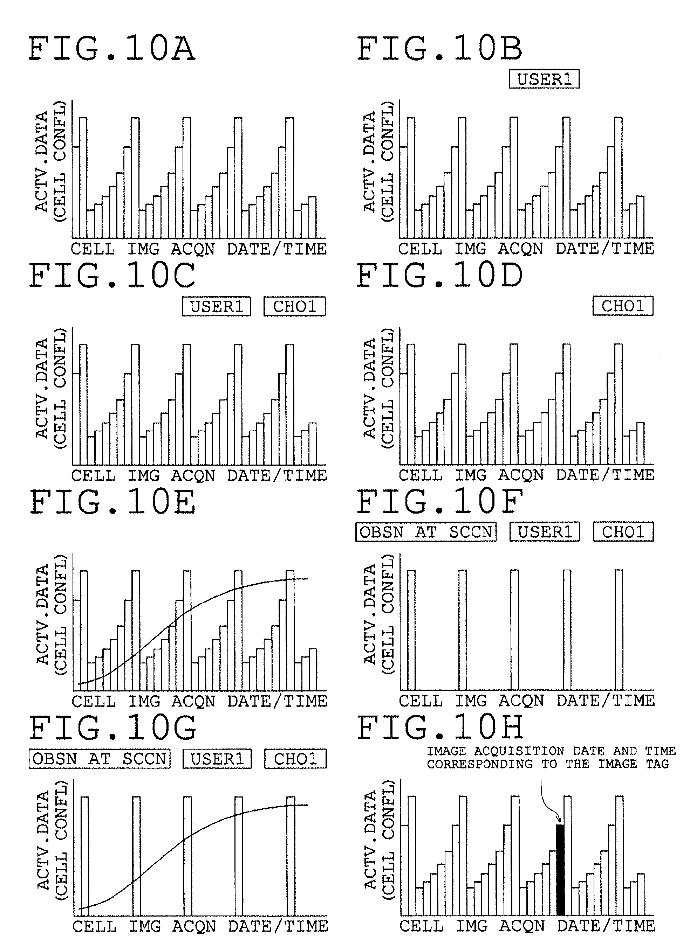

The data containing activity data provided in the search-tagged observation information set having been retrieved by the retrieving section 15 are displayed on a display surface (not shown in the drawings) in a display mode that depends on the search criterion generated by the search criterion generating section 17. The user checks cell quality by observing the data displayed on the display surface for example in a display mode as shown in FIGs. 10A-10G. Whereby, the procedure at the stage of searching and retrieving desired observation information sets on cells from the archive section and checking the cell quality is completed.

FIGs. 10A-10G are graphs that show, as examples of display modes on the display screen for presenting before the user data containing activity data provided in the observation information sets having been retrieved, out of the data containing activity data provided in the retrieved observation information sets at individual time points, cell confluency as activity data (bar graphs) and/or cumulated number of times of cell division (line graphs) calculated out on the basis of number of cells measured at each time point, plotted in bulk in order of date/time at which the observation information sets were acquired over several generations. The display surface shown in FIGs. 10A-10G may be configured so that the screen is switchable to a desired display mode by user operation. The display screens shown in FIGs. 10A-10G are mere examples of display mode of the display screen for presenting data containing retrieved observation information sets before users, and thus are not limited to these display modes, as a matter of course.

According to the cell observation information processing system 10 of the first embodiment mode, since the configuration is made so that the search criterion generating section 17 searches through the archive section 13 using the image tag selected by the user via the image selecting section 16, retrieves a search-tagged observation information set to which a search tag matched with the image tag has been assigned, and automatically generates a search criterion on the basis of subordinate tags, out of the search tags assigned to the search-tagged observation information set having been retrieved, and so that the retrieving section 15 searches through the archive section 13 using the search criterion generated by the search criterion generating section 17 and retrieves, from the archive section 13, data containing activity data provided in search-tagged observation information sets to which search tags matched with the search criterion have been assigned, only by an operation for selecting an image tag in the image selecting section 16, the user can set, in bulk, a plurality of information items that are to be search tags, as subordinate tags subordinately associated with the image tag; it is not necessary to set a plurality of tags one by one as in the case where the configuration described in Patent Document 1 is adopted. In addition, since activity data provided in the observation information sets are retrievable on the basis of subordinate tags, the user can be saved from the trouble of calculating activity data each time an image is retrieved as in the case where the configuration described in Patent Document 1 is adopted. The retrieved activity data can be graphed and displayed on a display surface upon being rearranged in order of date/time when the observation information sets were acquired. By making the display surface chronologically display the activity data, the user can instantly grasp the chronological variation of the activity data in association with the cell image indicated by the image tag selected from the image selecting section.

Therefore, according to the cell observation information processing system 10 of the first embodiment mode, the user is enabled to shorten the time taken to chronologically graph activity data of cells under culture by saving operation effort as much as possible, and is allowed to make intuitive operation without operation errors.

While the embodiment mode of the cell information processing system according to the present invention is explained above, the cell information processing system may be configured of a cell observation information processing program that makes a computer provided in the installation 1 function as: a storing means that assigns, to each of observation information sets each of which has been acquired via an observation information acquiring section in response to an acquisition order at each time point to carry a cell observation result such as a cell image, activity data indicating cell activity by a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date/time information for identifying a date/time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section; a search criterion generating means that lets a user select a desired image tag, searches through the archive section using the selected image tag, retrieves a search-tagged observation information set to which a search tag matched with the image tag has been assigned, and automatically generates a search criterion on the basis of subordinate tags, out of the search tags assigned to the search-tagged observation information set having been retrieved; and a retrieving means that searches through the archive section using the search criterion generated by the search criterion generating means and retrieves, from the archive section, data containing activity data provided in search-tagged observation information sets to which search tags matched with the search criterion have been assigned.

Also, the cell observation information processing system according to the embodiment mode of the present invention may have, other than the configuration in which a cell observation information processing program is provided in a computer provided in the installation 1, for example a configuration as recorded in a recording medium readable by a computer provided in the installation 1.

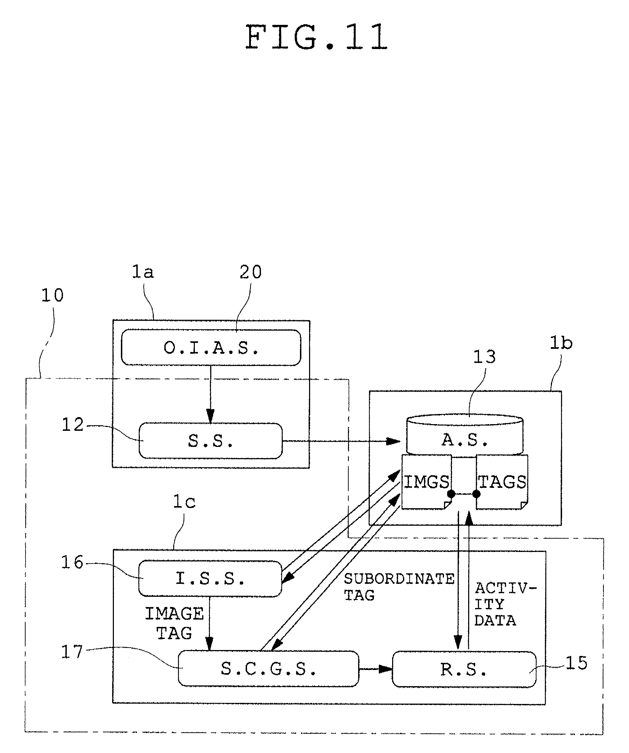

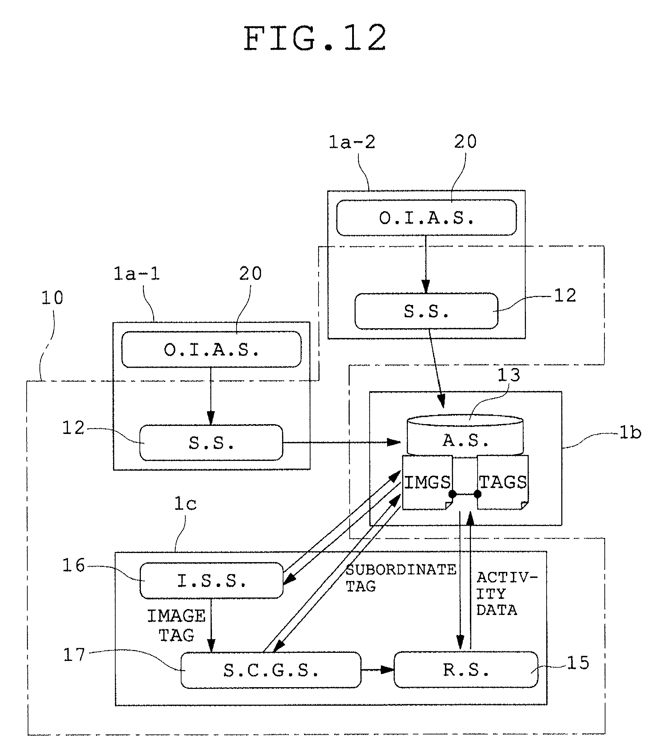

The cell observation information processing system according to the embodiment mode of the present invention is not limited to the configuration in which the storing section 12, the image selecting section 16, the search criterion generating section 17 and the retrieving section 15 are provided in one installation 1 as shown in FIG. 2, but may be configured so that, for example as shown in FIGs. 11 and 12 as modified examples, the storing section 12, the image selecting section 16, the search criterion generating section 17 and the retrieving section 15 are provided as distributed among a plurality of apparatuses. Also, the cell observation information processing system according to another embodiment mode of the present invention may be configured to include the archive section 13 also together with the storing section 12, the image selecting section 16, the search criterion generating section 17 and the retrieving section 15.

FIG. 11 is an explanatory diagram that shows the configuration of the cell observation information processing system according to one modified example of FIG. 2.

The cell observation information processing system 10 is configured so that the storing section 12 is provided in an observation processing apparatus 1a, the archive section 13 is provided in an archive processing apparatus 1b, and the image selecting section 16, the search criterion generating section 17 and the retrieving section 15 are provided in a search processing apparatus 1c. The observation information acquiring section 20 is provided in the observation processing apparatus 1a.

The observation processing apparatus 1a is configured, for example of a microscope apparatus provided with an image capture apparatus and a computer.

The archive processing apparatus 1b is configured, for example of a storage unit that stores database files.

The search processing apparatus 1c is configured, for example of a computer provided with a database software.

The observation processing apparatus 1a and the archive processing apparatus 1b, and the archive processing apparatus 1b and the search processing apparatus 1c are interconnected in network, respectively.

The configurations of the storing section 12, the archive section 13, the image selecting section 16, the search criterion generating section 17, the retrieving section 15 and the observation information acquiring section 20 are substantially the same as the configurations in the first embodiment mode shown in FIG. 2.

In the cell observation information processing system 10 of FIG. 11 thus configured, at the stage of storing observation information sets on cells under culture into the archive section 13, the observation information acquiring section 20 provided in the observation processing apparatus 1a acquires an observation information set in response to an acquisition order at one time point by a user. Then, a tag acquiring section (not shown in the drawings) acquires search tags for searching for the observation information set acquired via the observation information acquiring section 20. Then, the storing section 12 assigns, to the observation information set, as search tags for example including those acquired by the tag acquiring section (not shown in the drawings), an image tag, which forms a main tag, and information containing at least one of user identifying information, cell identifying information, date/time information, information on user's work for cell maintenance, apparatus identifying information, and activity data variation information, which form subordinate tags subordinately associated with the image tag, and then stores the search-tagged observation information set, into the archive section 13 in the archive apparatus 1b via a network line.

At the stage of searching and retrieving desired observation information set on cells from the archive section 13 and checking cell quality, the image selecting section 16 in the search processing apparatus 1c lets the user select a desired tag in the image tag selecting screen as shown in FIG. 5. Then, the search criterion generating section 17 searches through the archive section 13 via the network line using the image tag selected by the user in the image tag selecting screen, retrieves a search-tagged observation information set to which a search tag matched with the image tag is assigned, and automatically generates a search criterion on the basis of subordinate tags subordinately associated with the image tag, out of the search tags assigned to the search-tagged observation information set having been retrieved. Then, the retrieving section 15 searches through the archive processing apparatus 1b via the network line using the search criterion automatically generated by the search criterion generating section 17 and retrieves data containing activity data provided in search-tagged observation information sets to which search tags matched with the search criterion are assigned. The retrieved data containing search-tagged observation information sets are displayed on a display unit not shown in the drawings.

According to the cell observation information processing system of FIG. 11, since the storing section 12, the image selecting section 16, the search criterion generating section 17 and the retrieving section 15, which constitute the cell observation information processing system 10, are dividedly provided in a plurality of apparatuses 1a, 1b and 1c, the individual apparatuses can be made small-sized and simple.

Since the storing section 12 and the image selecting section 16, the search criterion generating section 17 and the retrieving section 15 are provided in the separate apparatuses 1a and 1c, the user can use different operation sites at the stage of storing observation information sets on cells under culture into the archive section and at the stage of searching and retrieving desired observation information sets on cells from the archive section 13 and checking cell quality, to enjoy improved convenience. For example, by using a mobile apparatus for the search processing apparatus 1c, without being tied to the location of the observation information acquiring apparatus 1a, the user can easily search and retrieve data containing desired observation information sets from the archive section 13 via network communication, to check cell quality.

It is noted that the storing section 12 may be provided in an apparatus different from the observation information acquiring section 20.

If the storing section 12 is provided in an apparatus different from the observation information acquiring section 20, the user can use different operation sites at the observation information acquiring stage and at the tag acquiring stage, to enj oy much improved convenience. For example, by using a mobile apparatus for the apparatus provided with a tag acquiring section and the storing section 12, without being tied to the location of the observation information acquiring apparatus 1a, the user can easily assign desired search tags to the observation information set acquired by the observation information acquiring section 20 and record them in the archive section 13 via network communication, by making the mobile apparatus display a search tag input screen (not shown in the drawings) and inputting the desired search tags.

It is noted that the image selecting section 16, the search criterion generating section 17 and the retrieving section 15 may be provided integrally in the observation processing apparatus 1a.

FIG. 12 is an explanatory diagram that shows the configuration of the cell observation information processing system according to another modified example of FIG. 2.

The cell observation information processing system 10 of this modified example is configured so that a plurality of observation processing apparatuses 1a-1 to 1a-n (in FIG. 12, n = 2 for convenience' sake) acquire observation information sets, and the observation information sets acquired by the respective observation processing apparatuses 1a-1 to 1a-n are managed in bulk.

To be specific, in the cell image observation processing system 10 of FIG. 12, each of the plurality of observation processing apparatuses 1a-1 to 1a-n is provided with an observation information acquiring section 20 and a storing section 12.

The other configuration is substantially the same as in the case of the cell information processing system 10 of FIG. 11.

In the cell observation information processing system 10 of FIG. 12 thus configured, at the stage of storing observation information sets on cells under culture into the archive section, in each of the observation processing apparatuses 1a-1 to 1a-2, the observation information acquiring section 20 acquires an observation information set in response to an acquisition order by a user at one time point, the tag acquiring section 11 acquires search tags for searching for the observation information set acquired via the observation information acquiring section 20, and the storing section 12 assigns the search tags acquired by the tag acquiring section to the observation information set and stores the search-tagged observation information set into the archive section 13 in the archive apparatus 1b via each network line.

The processing procedure at the stage of searching and retrieving desired observation information sets on cells from the archive section 13 and checking cell quality is substantially the same as that in the cell information processing system 10 of FIG. 11.

According to the cell observation information processing system 10 of FIG. 12, since the configuration is made so that a plurality of observation processing apparatuses 1a-1 to 1a-n (in FIG. 12, n = 2 for convenience' sake) acquire observation information sets and the observation information sets acquired by the respective observation processing apparatuses 1a-1 to 1a-n are managed in bulk, without being tied to the location of a particular observation processing apparatus 1a-n, a user can acquire an observation information set via an observation processing apparatus provided at a desired location and centrally record search-tagged observation information sets acquired via the individual observation processing apparatuses into the single archive section 13, to enjoy much improved convenience.

Further, since the configuration is made so that the observation data acquired by the plurality of observation processing apparatuses 1a-1 to 1a-n are managed in bulk, if each of the observation processing apparatuses is configured to be small-sized and light-weighted as a mobile unit for exclusive use by a particular user, each user can acquire observation information sets on cells at anyplace, to enj oymuch improved convenience.

The other configuration and function and effect are substantially the same as in the case of the modified example of FIG. 11.

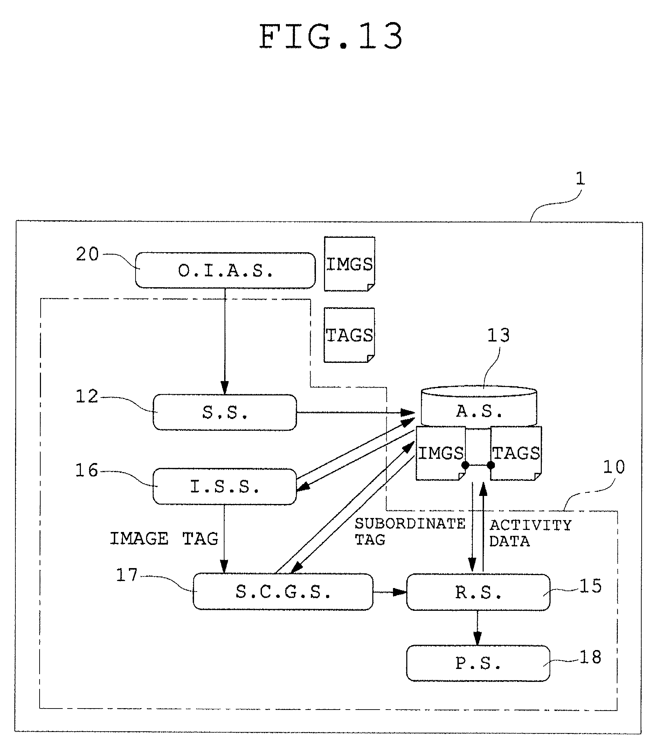

FIG. 13 is an explanatory diagram that shows the configuration of the cell observation information processing system according to still another modified example of FIG. 2.

According to the cell observation information processing system 10 of this modified example, the cell observation information processing system of FIG. 2 is configured to further include a presenting section 18.

The presenting section 18 creates a graph of data containing activity data provided in the search-tagged observation information sets retrieved by the retrieving section upon rearranging them chronologically on the basis of date/time when the observation information sets were acquired, and displays the graph as shown in FIG. 10H for example, on a display surface such as a monitor.

The other configuration is substantially the same as the configuration of the first embodiment mode shown in FIG. 2.

According to the cell observation information processing system 10 of FIG. 13, since a chronological graph of activity data on cells under culture is displayed in a short time without trouble only by selection of an image tag, the user can instantly grasp the conditions of chronological variation of the activity data.

It is preferred that the presenting section 18 is provided with a distinguishing display processing part for displaying, among a plurality of activity data sets to be presented, the activity data of the date/time when the observation information set corresponding to the selected image tag was acquired in a display manner different from activity data of the date/time when the remaining observation information sets were acquired. Display manners by the distinguishing display processing part preferably include, for example the emphasized display manner or the marker-overlaid display manner shown in FIG. 10H. In each of the display modes shown in FIGs. 10B-10G, the presenting section may be provided with the distinguishing display processing part, to make emphasized display or marker-overlaid display as shown in FIG. 10H, as a matter of course.

If such a distinguishing display processing part is provided, the position of the activity data of the date/time when the observation information set corresponding to the user-selected image tag was acquired is made conspicuous in a graph that chronologically shows activity data of a plurality of time points, for example. Therefore, in a situation where the presenting section is to display the cell image corresponding to the image tag along with the chronological graph of the activity data, the user can easily check the cell image and the activity data as associating them with each other.

According to the embodiment mode above, a captured image of a region about one local point (observation position) on a specimen containing cells is acquired. On the other hand, the system may be configured, as one modified example, to acquire, upon providing a plurality of cameras, a plurality of images by capturing images of a plurality of regions about respective local points (observation positions) at one time point using the plurality of cameras, and to treat the plurality of images acquired at one time point as one image group. In this case, one image tag and subordinate tags (regarding user ID, cell name etc.) subordinately associated with the image tag are assigned not to each image but to one image group.

While the configuration is made to select an image tag by user's manual operation according to the embodiment mode above, image tag selection may be configured as shown in the following modified example. Modified example 1: configuration in which image tag selection is made via alternative input mode by user

The image selecting section may be configured so that image tag selection is made via sound input, eye-gaze input or gesture input by a user.

For example in the case of voice input, the image selecting section may be configured to convert voice information that has been vocally inputted by a user into text information using voice analyzing technique as disclosed in JP KOKAI No. 2012-252181, and to select an image tag on the basis of the text information.

Also, for example in the case of eye-gaze input, the configuration may be made so that, upon correspondence information, in which moving directions of user's eye gaze are associated with certain letters, being stored in the system, the image selecting section detects directions of user's eye-gaze with an eye-gaze detecting section such as an eye-gaze detecting sensor, and selects an image tag corresponding to letters associated with the detected directions, on the basis of the detected directions of user's eye-gaze and the correspondence information.

Further, for example in the case of gesture input, the configuration may be made so that, upon correspondence information, in which moving directions of user's body parts such as limbs and trunk are associated with certain letters, being stored in the system, the image selecting section detects moving directions of user's body parts with a gesture detecting section such as a gesture detecting sensor, and selects an image tag corresponding to letters associated with the detected directions, on the basis of the detected moving directions of user's body parts and the correspondence information.

In this modified example, while examples of alternative input mode by user such as voice input and eye-gaze input are given, input mode by user is not limited to these. For example, the configuration may be made so that an image tag is acquired via input by brain waves.

In this way, according to the several embodiment modes of the present invention, it is possible to provide a cell observation information processing system, a cell observation information processing method, a cell observation information processing program, an archive section provided for the cell observation information processing system, and apparatuses provided for the cell observation information processing system, each of which enables users to shorten the time taken to chronologically graph activity data on cells under culture by saving operation effort as much as possible.

Alternatively, according to the several embodiment modes of the present invention, it is possible to provide a cell observation information processing system, a cell observation information processing method, a cell observation information processing program, an archive section provided for the cell observation information processing system, and apparatuses provided for the cell observation information processing system, each of which allows users to make intuitive operation, upon preventing operation errors in chronologically graphing activity data of cells under culture.

While the above explanation is made on the embodiment mode and its modified examples of the present invention, the present invention is not limited to the exact configuration as described in the embodiment mode and its modified examples. At the stage of reduction into practice, components can be embodied as being modified within the scope of not changing the essential concept of the invention. In addition, by appropriately combining a plurality of components described in the embodiment mode and its modified examples together, various inventions can be introduced. For example, some components may be removed from all of the components described in the embodiment mode and its modified examples or components described in different embodiment mode and its modified examples can be appropriately combined together. In this way, various modifications and applications are possible within the scope of not changing the essential concept of the invention.

### INDUSTRIAL APPLICABILITY

The cell observation information processing system, the cell observation information processing method, the cell observation information processing program, the archive section provided for the cell observation information processing system, and the apparatuses provided for the cell observation information processing system are useful, other than in the fields where there is a demand for quality management of cells used for research of biological object upon use of observation information on the cells acquired by an observation information acquiring apparatus such as a microscope, in fields where there is a demand for quality management of biological object that changes as time passes.

### DESCRIPTION OF THE REFERENCE SYMBOLS

- 1: cell observation information processing installation
- 1a, 1a-1, 1a-2: observation processing apparatus
- 1b: archive processing apparatus
- 1c: search processing apparatus
- 10: cell observation information processing system
- 12: storing section
- 13: archive section
- 15: retrieving section
- 16: image selecting section
- 17: search criterion generating section
- 18: presenting section
- 20: observation information acquiring section
- 34-1 to 34-n: image tags

## Claims

1. a cell observation information processing system comprising:
a storing section that assigns, to each of observation information sets each of which has been acquired via an observation information acquiring section in response to an acquisition order at each time point to carry a cell observation result including at last one of a cell image, activity data indicating cell activity by at least one of a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date and time information for identifying a date and time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section;
an image selecting section that lets a user select a desired image tag;
a search criterion generating section that searches through the archive section using the image tag selected via the image selecting section, retrieves a search-tagged observation information set to which a search tag matched with the image tag has been assigned, and automatically generates a search criterion on a basis of subordinate tags, out of the search tags assigned to the search-tagged observation information set having been retrieved; and
a retrieving section that searches through the archive section using the search criterion generated by the search criterion generating section and retrieves, from the archive section, data containing activity data provided in search-tagged observation information sets to which search tags matched with the search criterion have been assigned.

2. The cell observation information processing system according to claim 1,
**characterized in that**:
the search criterion generating section automatically generates, as the search criterion, information at least containing the cell identifying information.

3. The cell observation information processing system according to claim 2,
**characterized in that**:
the search criterion generating section automatically generates, as the search criterion, the information further containing the user identifying information.

4. The cell observation information processing system according to claim 3,
**characterized in that**:
the cell identifying information contains at least one of a cell name, a cell kind, a cell level information for distinguishing whether cells are in a primary culture to be cultured or in a subculture separated and cut out from the primary culture, and a passage number of cells, and
the search criterion generating section automatically generates, as the search criterion, information containing the user identifying information and at least one of the cell name, the cell kind, the cell level information and the passage number.

5. The cell observation information processing system according to claim 4,
**characterized in that**:
the search criterion generating section automatically generates, as the search criterion, information containing the user identifying information and at least one of the cell name and the cell kind.

6. The cell observation information processing system according to claim 5,
**characterized in that**:
the search criterion generating section automatically generates, as the search criterion, information containing the user identifying information, at least one of the cell name and the cell kind, and the cell level information.

7. The cell observation information processing system according to claim 6,
**characterized in that**:
the search criterion generating section automatically generates, as the search criterion, information containing the user identifying information, at least one of the cell name and the cell kind, the cell level information, and the passage number.

8. The cell observation information processing system according to claim 3,
**characterized in that**:
the search criterion generating section automatically generates, as the search criterion, information containing the user identifying information, the cell identifying information and the activity data variation information.

9. The cell observation information processing system according to claim 1,
**characterized in that**:
the archive section accommodates search-tagged observation information sets to each of which search tags adapted for cell observation directed to a plurality of types of cells are assigned, and
the search criterion generating section automatically generates, as the search criterion, information containing the cell identifying information and the activity data variation information.

10. The cell observation information processing system according to claim 1,
**characterized in that**:
the cell identifying information contains at least one of a cell name, a cell kind, cell level information for distinguishing whether cells are in a primary culture to be cultured or in a subculture separated and cut out from the primary culture, and a passage number of cells.

11. The cell observation information processing system according to claim 10,
**characterized in that**:
the search criterion generating section automatically generates, as the search criterion, information containing at least one of the cell name and the cell kind, and at least one of the cell level information and the passage number.

12. The cell observation information processing system according to claim 1,
**characterized in that**:
each of the observation information sets is constructed of information that carries a plurality of observation results corresponding to image capture at one time point directed to a plurality of observation positions on a specimen containing the cells.

13. The cell observation information processing system according to claim 12,
**characterized in that**:
the information carrying the plurality of observation results contains a plurality of cell images.

14. The cell observation information processing system according to claim 12,
**characterized in that**:
the information carrying the plurality of observation results contains a value calculated out on a basis of the plurality of cell images.

15. The cell observation information processing system according to claim 12,
**characterized in that**:
the information carrying the plurality of observation results contains a plurality of cell images and a value calculated out on a basis of the plurality of cell images.

16. The cell observation information processing system according to claim 12,
**characterized in that**:
the information carrying the plurality of observation results contains a plurality of cell images, and
the search tags include a value calculated out on a basis of the plurality of cell images.

17. The cell observation information processing system according to claim 12,
**characterized in that**:
the information carrying the plurality of observation results contains a plurality of cell images, and
the search tags include an image tag for indicating the plurality of cell images.

18. The cell observation information processing system according to claim 12,
**characterized in that**:
the information carrying the plurality of observation results contains a value calculated out on a basis of a plurality of cell images, and
the search tags include the value calculated out on a basis of the plurality of cell images.

19. The cell observation information processing system according to claim 12,
**characterized in that**:
the information carrying the plurality of observation results contains a plurality of cell images, and
the search tags include an image tag for indicating the plurality of cell images and a value calculated out on a basis of the plurality of cell images.

20. A cell observation information processing method comprising:
a storing step for assigning, to each of observation information sets each of which has been acquired via an observation information acquiring section in response to an acquisition order at each time point to carry a cell observation result including at least one of a cell image, activity data indicating cell activity by at least one of a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date and time information for identifying a date and time when the observation information set was acquired, information on user's work for cell maintenance, apparatus identifying information for identifying an apparatus used in acquiring the observation information set, and activity data variation information, which form subordinate tags subordinately associated with the image tag, and storing each of search-tagged observation information sets, to each of which the search tags have been assigned, into an archive section;
a search criterion generating step for letting a user select a desired image tag, searching through the archive section using the selected image tag, retrieving a search-tagged observation information set to which a search tag matched with the image tag has been assigned, and automatically generating a search criterion on a basis of subordinate tags, out of the search tags assigned to the search-tagged observation information set having been retrieved; and
a retrieving step for searching through the archive section using the search criterion generated at the search criterion generating step and retrieving, from the archive section, data containing activity data provided in search-tagged observation information sets to which search tags matched with the search criterion have been assigned.

21. A cell observation information processing program, **characterized by** making a computer function as:
a storing means that assigns, to each of observation information sets each of which has been acquired via an observation information acquiring section in response to an acquisition order at each time point to carry a cell observation result including at least one of a cell image, activity data indicating cell activity by at least one of a number of cells, a cell confluency, a morphology, and a survival rate, and an observation title, search tags for searching for the observation information set, the search tags including an image tag, which forms a main tag, for indicating the cell image, and information containing at least one of user identifying information for identifying a user who conducts cell observation, cell identifying information for identifying cells under observation, date and time information for identifying a date and time when the observation information set was acquired, information on user's work for cell maintenance, apparatus identifying information for identifying an apparatus used in acquiring the observation information set, and activity data variation information, which form subordinate tags subordinately associated with the image tag, and stores each of search-tagged observation information sets, to each of which the search tags have been assigned, into an archive section;
a search criterion generating means that lets a user select a desired image tag, searches through the archive section using the selected image tag, retrieves a search-tagged observation information set to which a search tag matched with the image tag has been assigned, and automatically generates a search criterion on a basis of subordinate tags, out of the search tags assigned to the search-tagged observation information set having been retrieved; and
a retrieving means that searches through the archive section using the search criterion generated by the search criterion generating means and retrieves, from the archive section, data containing activity data provided in search-tagged observation information sets to which search tags matched with the search criterion have been assigned.

22. The archive section provided for the cell observation information processing system according to claim 1,
**characterized in that**:
the archive section has one or more records of the search-tagged observation information set,
each of the records is created in response to image capture of cells at one time point, and
the observation information set corresponding to the image capture of cells at one time point and the search tags for searching for the observation information set are interrelated.

23. A plurality of apparatuses provided for the cell observation information processing system according to claim 1,
**characterized in that**:
the storing section, the image selecting section, the search criterion generating section and the retrieving section are separately arranged in the plurality of the apparatuses.

24. The cell observation information processing system according to claim 1,
**characterized in that**:
the cell observation information processing system further comprises a presenting section that presents to a user data containing activity data provided in the search-tagged observation information sets retrieved by the retrieving section upon rearranging the data chronologically on a basis of date and time when the observation information sets were acquired.

25. The cell observation information processing system according to claim 24,
**characterized in that**:
the presenting section includes a distinguishing display processing part that displays, among a plurality of sets of activity data to be presented, activity data of a date and time when the observation information set corresponding to the selected image tag was acquired in a display manner different from activity data of date and time when remaining observation information sets were acquired.
